# EUROPÄISCHE PATENTSCHRIFT

(11) **EP 0 714 661 B1**
(45) Veröffentlichungstag und Bekanntmachung des Hinweises auf die Patenterteilung: **11.02.1998**
(21) Anmeldenummer: 95118031.4
(22) Anmeldetag: 16.11.1995
(51) Int. Cl.: A61K 31/535, A61K 9/20

(54) **Verfahren zur Herstellung Molsidomin-haltiger Tabletten**
Process for the preparation of tablets containing molsidomine
Procédé de préparation de comprimés contenant de la molsidomine

(30) Priorität: 03.12.1994 DE 4443105
(43) Veröffentlichungstag der Anmeldung: 05.06.1996
(73) Patentinhaber: HOECHST AKTIENGESELLSCHAFT, 65926 Frankfurt am Main (DE)
(72) Erfinder: Voegele, Dieter, Dr., D-63486 Bruchköbel (DE)

(56) Entgegenhaltungen:
- EP-A- 0 624 370
- DE-A- 3 346 638

## Beschreibung

Die vorliegende Erfindung betrifft ein Verfahren zur Herstellung Molsidomin-haltiger retard-Tabletten.

Molsidomin (N-Ethoxycarbonyl-3-morpholino-sydnonimin) ist ein wertvoller pharmakologischer Wirkstoff zur Behandlung koronarer Herzerkrankungen und seit vielen Jahren im Handel.

Nachteilig ist allerdings, daß Molsidomin relativ instabil, insbesondere gegen Temperatur- und Feuchtigkeitseinflüsse aber auch gegen Lichteinwirkung ist. Bereits 1971 konnten Glykolsäure, Chloressigsäure, Morpholin, Ammoniumchlorid, Ethanol, Kohlendioxid und Stickstoff als Zersetzungsprodukte identifiziert werden (Asahi et al, Chem. Pharm. Bull. 19, 1079 (1971)). Die genannte Instabilität führt zu erheblichen Gehaltsminderungen entsprechender galenischer Zubereitungen, die beispielsweise bei Tabletten nach einer 72-stündigen Bestrahlung mit Metallhalogenid-Strahlern (entspricht einer Lagerung von etwa 4 bis 6 Wochen am Tageslicht) bis zu 60 % betragen kann (Thoma, Kerker, Pharm.Ind. 54, Nr.7, 630 (1992)).

Es hat deshalb nicht an Versuchen gefehlt, Molsidomin in Tabletten, beispielsweise durch entsprechende Verpackungen oder den Zusatz von Stabilisatoren, wie Farbstoffen und UV-Absorbern, zu stabilisieren. Allerdings ist eine überzeugende und endgültige Lösung des Problems noch nicht vorgeschlagen worden.

Bei der Herstellung von retard Tabletten ist es allgemein üblich, Wirkstoff und Hilfsstoffe zu mischen, zu granulieren und nach Bestäuben mit einem Formtrennmittel die erhaltene Tablettenmasse zu Tabletten zu verpressen.

Es wurde nun überraschenderweise gefunden, daß die Langzeitstabilität des Molsidomins in retard-Tabletten erheblich verbessert werden kann, wenn es mit einem vorgefertigten wirkstoffreien Trägergranulat vermischt und zu Tabletten verpreßt wird.

Die vorliegende Erfindung betrifft demnach ein Verfahren zur Herstellung Molsidomin-haltiger retard-Tabletten, dadurch gekennzeichnet, daß das Molsidomin mit einem wirkstoffreien Trägergranulat vermischt und zu Tabletten verpreßt wird.

Retard-Tabletten sind im Rahmen vorliegender Erfindung Tabletten, die ihren Wirkstoff verlängert in einem Zeitraum von 3 bis 24 Stunden nach Einnahme freisetzen.

Das erfindungsgemäße Verfahren wird bevorzugt bei Raumtemperatur durchgeführt. Das wirkstoffreie Trägergranulat besteht bevorzugt aus Füllmittel, Retardierungsmittel und Bindemittel, die in bei der Herstellung von retardTabletten gemäß Stand der Technik üblichen Mengen eingesetzt werden.

Beispielsweise enthält eine Molsidomin-haltige retard-Tablette 45 bis 80 Gew.% Füllmittel, 10 bis 30 Gew.% Retardierungsmittel und 5 bis 20 Gew.% Bindemittel, jeweils bezogen auf das Gesamttablettengewicht.

Beispiele für Füllmittel sind insbesondere Calciumhydrogenphosphat-dihydrat, Calciumsulfat-dihydrat, Lactose, Lactose-monohydrat, Lactose-Cellulose-Kombination, Mannit, Rohrzucker, Calciumhydrogenphosphat-dihydrat, Faser-cellulose, mikrokristalline Cellulose oder Mischungen der genannten Substanzen.

Bevorzugte Füllmittel sind Lactose-monohydrat, mikrokristalline Cellulose und Calciumhydrogenphosphatdihydrat.

Beispiele für Retardierungsmittel sind insbesondere hydrierte Öle, wie z.B. hydriertes Erdnußöl, hydriertes Cocosöl und hydriertes Ricinusöl; Ester, insbesondere Mono-, Di- und Triglyceride von Fettsäuren, wie z.B. Glycerinmonostearatpalmitat, Glycerintristearatpalmitat und Glycerinmonoditristearatpalmitat;
Ester von gereinigten Montanwachssäuren, höhere Fett-und Wachssäuren, wie z.B. Stearinsäure, Palmitinsäure, Behensäure, Myristinsäure und gereinigte Montanwachssäuren;
höhere Fettalkohole, wie z.B. Laurylalkohol, 12-Hydroxystearylalkohol, Cetyl-, Stearyl, Myristyl, Arachyl-, Carnaubyl- und Ceryl-alkohol;
natürliche, halbsynthetische und synthetische Wachse, wie z.B. Bienenwachs, Carnaubawachs, Paraffinwachs, Vaselinwachs, Ozokerit, Ceresin, Walrat und Polyäthylen mit niedrigem Erweichungspunkt;
Polyacrylsäuren sowie deren Salze und Ester;
Cellulosederivate, wie z.B. Methylhydroxyethylcellulosen, Methylcellulosen, Isopropylmethylcellulosen, Hydroxypropylcellulosen, Hydroxyethylcellulosen, Hydroxypropylmethylcellulosen, Carboxymethylcellulosen sowie deren Salze und Ester;
Alginsäuren sowie deren Salze und Ester; Guar-Gum, Carragen, Carboxymethyldextrane und quervernetztes Polyvinylpyrrolidon.

Es können auch Mischungen der genannten Retardierungsmittel verwendet werden. Bevorzugte Retardierungsmittel sind hydriertes Ricinusöl, Ester von gereinigten Monatanwachssäuren und Glycerinmonoditristearatpalmitat.

Beispiele für Bindemittel sind Polyvinylpyrrolidon und Polyethylenglykole mit mittleren Molekulargewichten von 1000 bis 35000. Es können auch Mischungen der genannten Bindemittel verwendet werden. Polyethylenglykole sind bevorzugte Bindemittel.

Die wirkstoffreien Trägergranulate können in an sich bekannter Weise, beispielsweise Trocken- oder Schmelzgranulierung, aus Füll-, Retardierungs- und Bindemittel erhalten werden.

Es ist besonderes vorteilhaft, die durch Mischung des wirkstoffreien Trägergranulats mit dem Molsidomin erhaltene Tablettenmasse vor dem Verpressen zu Tabletten mit einem Formtrennmittel zu bestäuben. Geeignete Formtrennmittel sind beispielsweise Magnesiumstearat, Calciumstearat, Zinkstearat, Aluminiumstearat, Calciumbehenat, Talkum, Silikonöl und Natriumstearylfumarat, die in Mengen von 0,5 bis 3 Gew.%, bezogen auf die Tablettenmasse, zugegeben werden. Ein bevorzugtes Formtrennmittel ist Magnesiumstearat.

In einer besonderen Ausführungsform des erfindungsgemäßen Verfahrens wird Molsidomin in Mischung mit einem Füllmittel und/oder Bindemittel dem wirkstoffreien Trägergranulat zugemischt. Die Zusammensetzungen dieses wirkstoffreien Trägergranulats sowie der Molsidomin-Mischung werden dabei sinnvollerweise so gewählt, daß die gewünschte Endzusammensetzung der Tablette erreicht wird.

Die anspruchsgemäßen Tabletten enthalten Molsidomin bevorzugt in Mengen von 0,5 bis 30 mg/Tablette, besonders bevorzugt in Mengen von 1 bis 15 mg/Tablette und ganz besonders bevorzugt in Mengen von 6 bis 10 mg/Tablette.

Daneben können die anspruchsgemäßen Tabletten noch weitere Hilfsstoffe enthalten. Beispiele sind übliche Geschmacks-, Süß- und Aromamittel, Lebensmittelfarbstoffe, sowie Stabilisatoren, wie sie beispielsweise in EP-B 0 147 811 und EP-B 0 206 219 genannt sind. Ein bevorzugter Stabilisator ist Troxerutin.

Durch das erfindungsgemäße Verfahren wird im Vergleich zu den Verfahren des Standes der Technik das Freisetzungsprofil der retard-Tabletten nicht verändert, d.h. die Geschwindigkeit der Wirkstoffreisetzung bleibt unverändert.

### Beispiel 1

106 g Lactose-monohydrat, 42 g mikrokristalline Cellulose, 12 g eines festen Polyethylenglykols und 30 g hydriertes Ricinusöl werden unter Erwärmen gemischt, bis sich ein Granulat bildet. Dieses wird abgekühlt und durch ein Sieb passiert.
Diesem Trägergranulat werden 8 g Molsidomin zugemischt, die Tablettenmasse mit 2 g Magnesiumstearat bestäubt und zu Tabletten verpreßt.

### Beispiel 2

Beispiel 1 wird wiederholt mit dem Unterschied, daß das Trägergranulat unter Verwendung von nur 98 g Lactosemonohydrat hergestellt wird und dafür Molsidomin in Mischung mit 8 g Lactose-monohydrat dem abgekühlten und gesiebten Trägergranulat zugemischt wird.

### Beispiel 3

106 g Lactose-monohydrat, 42 g mikrokristalline Cellulose, 12 g eines festen Polyethylenglycols und 30 g hydriertes Ricinusöl werden gemischt und kompaktiert. Die Komprimate werden durch ein Sieb passiert.
Diesem Trägergranulat werden 8 g Molsidomin zugemischt, die Tablettenmasse wird mit 2 g Magnesiumstearat bestäubt und zu Tabletten verpreßt.

### Beispiel 4

Beispiel 3 wird wiederholt mit dem Unterschied, daß das Trägergranulat unter Verwendung von nur 98 g Lactosemonohydrat hergestellt wird und dafür Molsidomin in Mischung mit 8 g Lactose-monohydrat dem gesiebten Trägergranulat zugemischt wird.

### Beispiel 5

95 g Lactose-monohydrat, 40 g mikrokristalline Cellulose, 12 g eines festen Polyethylenglycols und 35 g hydriertes Ricinusöl werden unter Erwärmen gemischt, bis sich ein Granulat bildet. Dieses wird abgekühlt und durch ein Sieb passiert.
Diesem Trägergranulat werden 12 g Molsidomin in Mischung mit 4 g Lactose-monohydrat zugemischt, die Tablettenmasse wird mit 2 g Magnesiumstearat bestäubt und zu Tabletten verpreßt.

### Beispiel 6

Beispiel 5 wird wiederholt mit dem Unterschied, daß das Trägergranulat unter Verwendung von nur 94 g Lactosemonohydrat hergestellt wird, 12 g Molsidomin in Mischung mit 4 g Lactose-monohydrat dem abgekühlten und gesiebten Trägergranulat zugemischt werden, die Tablettenmasse mit 3 g Magnesiumstearat bestäubt und zu Tabletten verpreßt wird.

### Beispiel 7

100 g Lactose-monohydrat, 42 g mikrokristalline Cellulose, 18 g eines festen Polyethylenglycols, 20 g hydriertes Ricinusöl und 10 g Ester gereinigter Montanwachssäuren werden unter Erwärmen gemischt, bis sich ein Granulat bildet.
Diesem Trägergranulat werden 8 g Molsidomin zugemischt, die Tablettenmasse wird mit 2 g Magnesiumstearat bestäubt und zu Tabletten verpreßt.

### Beispiel 8

Beispiel 7 wird wiederholt mit dem Unterschied, daß das Trägergranulat unter Verwendung von nur 72 g Lactosemonohydrat, dafür aber 62 g mikrokristalliner Cellulose hergestellt wird und 8 g Molsidomin in Mischung mit 8 g Lactose-monohydrat dem gesiebten Trägergranulat zugemischt werden, die Tablettenmasse mit 2 g Magnesiumstearat bestäubt und zu Tabletten verpreßt wird.

### Beispiel 9

106 g Lactose-monohydrat, 35 g mikrokristalline Cellulose, 10 g eines festen Polyethylenglycols, 20 g hydriertes Ricinusöl und 10 g Glycerinmonoditristearatpalmitat werden unter Erwärmen gemischt, bis sich ein Granulat bildet. Dieses wird abgekühlt und durch ein Sieb passiert.
Diesem Trägergranulat werden 8 g Molsidomin in Mischung mit 8 g Lactose-monohydrat zugemischt, die Tablettenmasse wird mit 3 g Magnesiumstearat bestäubt und zu Tabletten verpreßt.

### Beispiel 10

Beispiel 9 wird wiederholt mit dem Unterschied, daß das Trägergranulat unter Verwendung von nur 73 g Lactose-monohydrat, dafür aber 64 g mikrokristalline Cellulose hergestellt wird, 10 g Molsidomin in Mischung mit 10 g Lactose-monhydrat dem abgekühlten und gesiebten Trägergranulat zugemischt werden, die Tablettenmasse mit 3 g Magnesiumstearat bestäubt und zu Tabletten verpreßt wird.

Zur Bestimmung der Langzeitstabilität des Molsidomins in den erfindungsgemäß hergestellten retard-Tabletten wurden gemäß Beispiel 2 hergestellte, 8 mg Molsidomin enthaltende Tabletten, 3, 6 und 12 Monate bei einer Temperatur von 40° C gelagert und anschließend deren Morpholingehalt bestimmt. Zum Vergleich wurden Tabletten identischer Zusammensetzung, aber durch gemeinsames Schmelzgranulieren aller Bestandteile (außer Formtrennmittel) hergestellte Tabletten herangezogen.

Dabei wurde der Morpholingehalt wie folgt bestimmt: Die Tablette wird pulverisiert und mit Wasser extrahiert. Die so erhaltene wäßrige Lösung wird durch Flüssigchromatographie (LC) gemäß Pharm. Eur. 2 (v.6.20.4) analysiert. Es wird eine Zweisäulentechnik (Suppressor-Technik) angewendet, wobei eine Dionex-CS-10-Säule und als mobile Phase eine Lösung von 7 ml 37 % Salzsäure und 1000 mg DL-2,3-Diaminpropionsäure in 2000 ml doppelt destilliertem Wasser zur Anwendung kommt. Zur Regenerierung des Suppressors wird eine Lösung von 300 ml 40 %igem Tetrabutylammoniumhydroxid in 5000 ml doppelt destilliertem Wasser verwendet. Die Detektion erfolgt über eine Leitfähigkeitsmessung. Die Bestimmungsgrenze dieser Methode liegt bei 100 mg Morpholin/kg Molsidomin.

Folgende Ergebnisse wurden erhalten:

| | Morpholin (mg/kg Molsidomin) | | |
|---|---|---|---|
| | 3 Monate | 6 Monate | 12 Monate |
| erfindungsgemäß | 190 | <100 | <100 |
| Stand der Technik | 290 | 350 | 590 |

## Patentansprüche

1. Verfahren zur Herstellung Molsidomin-haltiger retardTabletten, dadurch gekennzeichnet, daß das Molsidomin mit einem wirkstoffreien Trägergranulat vermischt und zu Tabletten verpreßt wird.

2. Verfahren gemäß Anspruch 1, dadurch gekennzeichnet, daß es bei Raumtemperatur durchgeführt wird.

3. Verfahren gemäß Anspruch 1 und/oder 2, dadurch gekennzeichnet, daß das wirkstoffreie Trägergranulat aus Füllmittel, Retardierungsmittel und Bindemittel besteht.

4. Verfahren gemäß Anspruch 3, dadurch gekennzeichnet, daß als Füllmittel Lactose-monohydrat, mikrokristalline Cellulose oder Calciumhydrogenphosphat-dihydrat eingesetzt wird.

5. Verfahren gemäß Anspruch 3, dadurch gekennzeichnet, daß als Retardierungsmittel hydriertes Ricinusöl, Ester von gereinigten Montanwachssäuren oder Glycerinmonoditristearatpalmitat eingesetzt wird.

6. Verfahren gemäß Anspruch 3, dadurch gekennzeichnet, daß als Bindemittel Polyethylenglykole eingesetzt werden.

7. Verfahren gemäß einem oder mehreren der Ansprüche 1 bis 6, dadurch gekennzeichnet, daß die durch Mischung des wirkstoffreien Trägergranulats mit dem Molsidomin erhaltene Tablettenmasse vor dem Verpressen zu Tabletten mit einem Formtrennmittel bestäubt wird.

8. Verfahren gemäß Anspruch 7, dadurch gekennzeichnet, daß als Formtrennmittel Magnesiumstearat eingesetzt wird.

9. Verfahren gemäß einem oder mehreren der Ansprüche 1 bis 8, dadurch gekennzeichnet, daß das Molsidomin in Mischung mit Füllmitteln und/oder Bindemitteln dem wirkstoffreien Trägergranulat zugemischt wird.

## Claims

1. A process for the preparation of delayed-release tablets containing molsidomine, which comprises mixing molsidomine with active compound-free carrier granules and compressing to give tablets.

2. The process as claimed in Claim 1, which process is carried out at room temperature.

3. The process as claimed in Claim 1 and/or 2, wherein the active compound-free carrier granules consist of filler, delayed-release agent and binder.

4. The process as claimed in Claim 3, wherein the filler employed is lactose monohydrate, microcrystalline cellulose or calcium hydrogen phosphate dihydrate.

5. The process as claimed in Claim 3, wherein the delayed-release agent employed is hydrogenated castor oil, esters of purified montan wax acids or glycerol monoditristearate palmitate.

6. The process as claimed in Claim 3, wherein the binders employed are polyethylene glycols.

7. The process as claimed in one or more of Claims 1 to 6, wherein the tablet material obtained by mixing the active compound-free carrier granules with the molsidomine is dusted with a mold-release agent before compressing to give tablets.

8. The process as claimed in claim 7, wherein the mold-release agent employed is magnesium stearate.

9. The process as claimed in one or more of Claims 1 to 8, wherein the molsidomine is admixed to the active compound-free carrier granules as a mixture with fillers and/or binders.

## Revendications

1. Procédé de préparation de comprimés à libération prolongée contenant de la molsidomine, caractérisé en ce que la molsidomine est mélangée à un granulat véhiculaire sans principe actif puis pressée en comprimés.

2. Procédé selon la revendication 1, caractérisé en ce qu'il est conduit à température ambiante.

3. Procédé selon les revendications 1 et/ou 2, caractérisé en ce que le granulat véhiculaire sans principe actif se compose d'un composé de charge, d'un agent de libération prolongée et d'un liant.

4. Procédé selon la revendication 3, caractérisé en ce que le composé de charge utilisé est du monohydrate de lactose, de la cellulose microcristalline ou du dihydrate d'hydrogénophosphate de calcium.

5. Procédé selon la revendication 3, caractérisé en ce que l'agent de libération prolongée utilisé est de l'huile de ricin hydrogénée, des esters d'acides purifiés de cire minérale ou du monoditristéarate palmitate de glycérol.

6. Procédé selon la revendication 3, caractérisé en ce que l'on utilise comme liant des polyéthylène glycols.

7. Procédé selon une ou plusieurs des revendications 1 à 6, caractérisé en ce que l'on répand un agent de démoulage sur la masse obtenue par mélange du granulat véhiculaire sans principe actif avec la molsidomine, avant le pressage de celle-ci en comprimés.

8. Procédé selon la revendication 7, caractérisé en ce que l'agent de démoulage utilisé est le stéarate de magnésium.

9. Procédé selon une ou plusieurs des revendications 1 à 8, caractérisé en ce que la molsidomine mélangée au composé de charge et/ou au liant est ajoutée par mélange au granulat véhiculaire sans principe actif.
